# EUROPEAN PATENT SPECIFICATION

(11) **EP 2 036 551 B1**
(45) Date of publication and mention of the grant of the patent: **06.11.2013**
(21) Application number: 07737277.9
(22) Date of filing: 12.06.2007
(51) Int. Cl.: A61K 31/353, A23L 1/30, A23L 2/38, A61P 21/00, A23L 1/00

(54) **CATECHINS FOR IMPROVING MUSCLE POWER**
KATECHINE ZUR VERBESSERUNG DER MUSKELKRAFT
CATÉCHINES POUR L'AMÉLIORATION DE LA PUISSANCE MUSCULAIRE

(30) Priority: 05.07.2006 JP 2006185452
(43) Date of publication of application: 18.03.2009
(73) Proprietor: Kao Corporation, Chuo-ku Tokyo 103-8210 (JP)
(72) Inventor: OTA, Noriyasu, Haga-gun, Tochigi 321-3497 (JP); MURASE, Takatoshi, Haga-gun, Tochigi 321-3497 (JP)
(74) Representative: HOFFMANN EITLE
(86) International application number: PCT/JP2007/000622
(87) International publication number: WO 2008/004333

(56) References cited:
- EP-A1- 1 671 963
- EP-A1- 1 813 156
- WO-A1-2005/028464
- WO-A1-2005/074962
- WO-A1-2006/051980
- WO-A1-2007/006135
- JP-A- 09 322 716
- JP-A- 2003 010 158
- JP-A- 2006 117 687
- US-A1- 2005 281 896
- SINGAL A ET AL: "Green Tea Extract and Catechin Ameliorate Chronic Fatigue-Induced Oxidative Stress in Mice" JOURNAL OF MEDICINAL FOOD, MARY ANN LIEBERT, LARCHMONT, NY, US LNKD- DOI:10.1089/JMF.2005.8.47, vol. 8, no. 1, 1 January 2005 (2005-01-01) , pages 47-52, XP003015493 ISSN: 1096-620X
- DORCHIES OLIVIER M ET AL: "Green tea extract and its major polyphenol (-)-epigallocatechin gallate improve muscle function in a mouse model for Duchenne muscular dystrophy" AMERICAN JOURNAL OF PHYSIOLOGY. CELL PHYSIOLOGY, AMERICAN PHYSIOLOGICAL SOCIETY, US LNKD- DOI:10.1152/AJPCELL.00425.2005, vol. 290, no. 2, 1 February 2006 (2006-02-01), pages C616-C625, XP002459514 ISSN: 0363-6143
- NAGASAWA T.: 'Undo ni yoru Stress ni Taisuru Cha-Catechin no Koka (EFFECTS OF THE CATECHIN ON EXERCISE-INDUCED OXIDATIVE STRESS)' BIOSCIENCE & INDUSTRY vol. 60, no. 3, 2002, pages 173 - 174, XP002991259
- DORCHIES OLIVIER M ET AL: "Green tea extract and its major polyphenol (-)-epigallocatechin gallate improve muscle function in a mouse model for Duchenne muscular dystrophy", AMERICAN JOURNAL OF PHYSIOLOGY. CELL PHYSIOLOGY, AMERICAN PHYSIOLOGICAL SOCIETY, US, vol. 290, no. 2, 1 February 2006 (2006-02-01), pages C616-C625, XP002459514, ISSN: 0363-6143, DOI: 10.1152/AJPCELL.00425.2005

## Description

### Technical Field

The present invention relates to a pharmaceutical product, a food and beverage and similar products which exhibit effect of improving muscle force.

### Background Art

Generally, physical exercise and well-balanced nutritional supply are considered important factors for improving motor performance including muscle force. Recently, athletes and people who regularly do physical exercise have come to take a nutritive supplement or the like in combination with physical training, for more effectively improving muscle performance (Patent Document 1).

Meanwhile, people who are concerned with their obesity sometimes take an excessively calorie-lowered diet and fail to take sufficient nutrients into their body, which lead to a problem that skeletal muscle and muscle force in such people are reduced. For non-obese people, improvement of muscle force is also preferred, from the viewpoints of mitigation of long-lasting fatigue and promotion of health.

Under such circumstances, there is demand for a method for effectively improving muscle force with safety among athletes and physical exercise-lovers who want to improve their motor performance, among those who want to mitigate long-lasting fatigue, obesity, etc., and among other people.

For satisfying the demand, screening of components exhibiting an effect for improving muscle force or inhibiting muscle fatigue has been carried out. For example, a composition containing arginine (Patent Document 2), a polymeric fruit polyphenol (Patent Document 3) or ornithine (Non-Patent Document 1) has been reported to have a function of improving muscle force.

Meanwhile, physiologically useful properties of catechins contained in green tea and the like have already been reported, such as cholesterol level increase suppression action (Patent Document 4), blood sugar level increase suppression action (Patent Document 5), physical endurance enhancing action (Patent Document 6), and muscular dystrophy suppression action (Non-Patent Document 2). However, hitherto, it has not been known about the effect of catechin on improving muscle force or inhibiting muscle fatigue.
Patent Document 1: JP-A-2002-065212
Patent Document 2: JP-A-2004-256513
Patent Document 3: WO 2005/074962, pamphlet
Patent Document 4: JP-A-S60-156614
Patent Document 5: JP-A-H4-253918
Patent Document 6: JP-A-2005-89384, corresponding to EP 1 671 963
Non-Patent Document 1: Elam R. P. et al, J. Sports Med & Phys Fitness, 52-6, 1989
Non-Patent Document 2: Dorchies O. M. et al, AJP-Cell Physiol, 616-25, 2006

WO 2007/006135 represents state of the art according to Article 54(3) EPC and describes a dietary supplement for preventing muscle protein degradation. The dietary supplement comprises a source of at least one of epigallocatechin gallate (EGCG), epicatechin gallate (ECG), epicatechin (EC), tannic acid or related catechins; and a source of gypenosides.

EP 1813153, representing state of the art according to Article 54(3) EPC, relates to packaged beverages for fat-burning acceleration, energy-consumption boosting or exercise-endurance enhancement. The beverages contain a green tea extract added therein and having a pH of from 2 to 7, and also containing the following ingredients (A) to (C): (A) from 0.01 to 1.0 wt% of non-polymer catechins, (B) from 0.001 to 0.5 wt% of sodium ions, and (C) from 0.001 to 0.2 wt% of potassium ions.

Signal et al. J. Med. Food 8(1), 2005, pages 47-52 address the chronic fatigue syndrome (CFS), which is an illness characterized by persistent and relapsing fatigue, often accompanied by numerous symptoms involving various body systems. The relationship between CFS and oxidative stress is examined in a mouse model. It is found that green tea extract and catechin ameliorate chronic fatigue-induced oxidative stress in mice.

US 2005/281896 is directed to a nutritional composition comprising aqueous solution of cinnamon and creatine. In addition, the nutritional composition may also include alpha lipoic acid and various additional ingredients such as green or white tea extracts or caffeine. The composition is said to promote creatine accumulation, build muscle size, increase thermogenesis, reduce body fat mass leading to weight loss and/or improving muscular definition.

### Disclosure of the Invention

Accordingly, the present invention is directed to the following:
(1) Non-medical use of catechins as effective ingredient in combination with physical exercise for improving muscle force, wherein the use of a dietary supplement comprising a source of at least one of epigallocatechin gallate (EGCG), epicatechin gallate (ECG), epicatechin (EC), tannic acid or related catechins and a source of Gypenosides is excluded.
(2) Non-medical use of catechins as effective ingredient for increasing the effect of physical exercise on the enhancement of muscle force, wherein the use of a dietary supplement comprising a source of at least one of epigallocatechin gallate (EGCG), epicatechin gallate (ECG), epicatechin (EC), tannic acid or related catechins; and a source of Gypenosides is excluded.
(3) Non-medical use according to item (1) or (2), wherein the catechins are selected from the group consisting of epicatechin gallate, gallocatechin gallate and epigallocatechin.

### Brief Description of the Drawings

[Fig. 1]
   A graph showing improvement of muscle force in mice after intake of catechin.
[Fig. 2]
   Effects of catechin species on fatigue resistance of isolated muscle.

### Detailed Description of the Present Invention

The present invention provides foods and beverages and similar products which have effects of improving muscle force and improving exercise effect with high safety.

The present inventors have carried out extensive studies on physiological actions of catechins, and surprisingly have found that catechins have an excellent effect of improving muscle force and improving exercise effect.

The agent for improving muscle force according to the present invention contains catechin, which has a long history as a foodstuff and therefore has high safety, as an effective ingredient. Thus, through use of these agents, improvement of muscle force can be attained with virtually no adverse side effects.

The term "catechin" in the present invention collectively refers to non-epi-catechins such as catechin, catechin gallate, gallocatechin, and gallocatechin gallate; and epi-catechins such as epicatechin, epigallocatechin, epicatechin gallate, and epigallocatechin gallate. Catechin in the present invention preferably contains at least one selected from the above catechin species, more preferably, catechin gallate, gallocatechin, gallocatechin gallate, epicatechin, epigallocatechin, epicatechin gallate or epigallocatechin gallate, still more preferably, epicatechin gallate, gallocatechin gallate or epigallocatechin gallate are employed. The catechin of the present invention is preferably a non-polymeric species.

Generally, the catechin employed in the present invention may be obtained through direct extraction of tea leaves or through concentrating or purifying the tea extract. Alternatively, the catechin may be obtained from other materials, or may be a column-purified or a chemically synthesized product.

In one embodiment of extraction of catechin from tea leaves, water or hot water, or water or hot water containing an extraction aid, was added to a processed tea leaf derived from tea leaf belonging to the genus *Camellia* (e.g., *C. sinensis* or *C. assamica* or a hybrid thereof) to prepare tea extract, through a known method such as extraction with stirring. The extraction may also be performed in a non-oxidizing atmosphere during which dissolved oxygen was removed through boiling deaeration or feeding of an inert gas (e.g., nitrogen) thereto.
The processed tea leaf includes (1) green teas such as Sen-cha, Ban-cha, Gyokuro, Ten-cha, and kamairi-cha; (2) semi-fermented teas which are so-called oolong teas, such as Ti guan yin, Sezhong, Huang jin gui, and Wu yi yan cha; and (3) fermented teas called black teas such as Darjeeling, Uva, and Keemun.
Examples of the extraction aid include organic acids and salts thereof such as sodium ascorbate.

The tea extract concentrate may be produced through concentration of the above-described extract, or the tea extract may be purified by use of a solvent or by means of a column. The concentrated or purified tea extract may be in the form of solid, aqueous solution, slurry, and etc.
For example, the tea extract may be prepared through methods disclosed in detail in, for example, JP-A-S59-219384, JP-A-H4-20589, JP-A-H5-260907 and JP-A-H5-306279. The tea extract may be a commercial product. Examples of the commercial product include POLYPHENON (product of Mitsui Norin Co., Ltd.), Teaflan (product of Ito En Ltd.), Sunphenon (product of Taiyo Kagaku Co., Ltd.), and Sunoolong (product of Suntory Limited).

The catechins contained in the tea extract are present in the form of non-polymeric species which is dissolved in the liquid, or in the form of solid which is adsorbed or included by tea micropowder suspended in the liquid.
The catechin content in the extract is 10 to 100 mass%, preferably 30 to 95 mass%, particularly preferably 40 to 80 mass%. The ratio of the amount of catechin to the total amount of polyphenols contained in the tea extract is 10 mass% or higher immediately after production, preferably 20 mass% or higher.
For more effectively attaining improvement of muscle force, the ratio of the amount of one or more species selected from among gallocatechin, epigallocatechin, catechin gallate, epicatechin gallate, gallocatechin gallate, and epigallocatechin gallate to the total amount of catechins contained in the tea extract is preferably 40% or more, more preferably 60% or more, and even more preferably 80% or more.

Most of the catechins contained in tea leaves are in the form of epi-catechin. The epi-catechin can be converted into the corresponding non-epi-catechin, which is a stereoisomer, through treatment with heat, acid, alkali, etc. Thus, when a non-epi-catechin is employed, an extract of green tea, semi-fermented tea or fermented tea, or a concentrate thereof is dissolved in water, and the aqueous solution is heated, for example, at 40 to 140°C for 0.1 minutes to 120 hours, to thereby obtain a non-epi-catechin. Alternatively, a concentrated tea extract having high non-epi-catechin content may also be employed. Epi-catechins and non-epicatechins may be used singly or in combination.

As described in the Examples herein below, catechin has an effect of improving muscle force in mice. When catechin is employed in combination with physical exercise, the effect of improving muscle force is more potentiated. Therefore, catechin can be employed as an agent for improving muscle force or for improving exercise effect. Catechin can be used for the production of an agent of improving muscle force or for improving exercise effect (hereinafter referred to as an agent for improving muscle force and etc.). The agent for improving muscle force and etc. are useful for producing foods and beverages for human and animals which are effective for improving muscle force during physical exercise. When the agent are used as foods and beverages, foods and beverages such as functional foods, which are consumed for improving physiological functions (e.g., maintaining or improving muscle force and improving exercise effect) can be provided.
The term "improving exercise effect" is referred to as an effect of improving enhancement of muscle force by exercise and making physical exercise more effectively.

The agent for improving muscle force and etc. of the present invention can be provided in the form of peroral solid preparations such as tablets and granules and peroral liquid preparations such as oral liquid and syrup may be provided.
For preparing peroral solid preparations, catechin is mixed with a vehicle and, if needed, optional additives such as a binder, a disintegrant, a lubricant, a colorant, a flavoring agent and a corrigent, and the mixture is processed through an ordinary method, to thereby produce tablets, coated tablets, granules, powders, capsules, and etc. For preparing peroral liquid preparations, catechin is mixed with additives such as a flavoring agent, a buffer, a stabilizer and a corrigent, and the mixture is processed through an ordinary method, to thereby produce oral liquid, syrup, elixir, and etc.

The agent for improving muscle force and etc. of the present invention can be incorporated into foods and beverages of any form. Examples include beverages, jelly, snacks, baked snacks, fried cakes, cakes, chocolate, gum, candies, soup, noodles and rice foods. Among them, beverages are preferred. For example, tea-based beverages such as Oolong tea, green tea and black tea; and non-tea-based beverages such as carbonated drinks (soft drinks), fruit extract-containing beverages, vegetable extract-containing juice, near-water, sport beverages, isotonic beverages and diet beverages may be provided. These beverages are preferably provided as container-packed beverages. As used herein, the term "tea-based beverage" refers to a beverage having characteristic tea flavor and taste, and the term "non-tea-based beverages" refers to beverages other than tea-based beverages. Instead of the pharmaceutical preparation, the corresponding tea extract may also be employed for producing such beverages. The tea extract may be dissolved in or diluted with, for example, water, carbonated water or generally employed tea extract liquid.

Examples of the container-packed beverages include a non-tea-based container-packed beverage (disclosed in Japanese Patent No. 3742094) and a tea-based container-packed beverage (disclosed in JP-A-2002-272373).
Formulation examples will next be described.

### A) Non-tea-based container-packed beverage

A container-packed beverage having a pH of 2 to 6 and containing the following ingredients (A) to (D):
(A) non-polymeric catechins: 0.01 to 1.0 wt.%,
(B) sweetener: 0.0001 to 15 wt.%,
(C) sodium ions: 0.001 to 0.5 wt.%, and
(D) potassium ions: 0.001 to 0.2 wt.%.

The sweetener (B) employed in the beverage is an artificial sweetener, a carbohydrate or a glycerol. Examples of the artificial sweetener include high-sweetness sweeteners such as aspartame, saccharin, cyclamate, acesulfame-K, L-aspartyl-L-phenylalanine lower alkyl ester sweetener, L-aspartyl-D-analinamide, L-aspartyl-D-serinamide, L-aspartyl-hydroxymethylalkanamide sweetener, L-aspartyl-1-hydroxyethylalkanamide sweetener, sucralose and thaumatin; sugar alcohols such as erythritol, xylitol and trehalose; glycyrrhizin; and synthetic alkoxy aromatic compounds. Natural sweeteners such as stevinoside may also be used.

An example of the carbohydrate sweetener employed in the beverage is a soluble carbohydrate. The soluble carbohydrate works as a sweetener and as an energy source. Examples of the carbohydrate include monosaccharides, oligosaccharides, complex polysaccharides, and mixtures thereof. Examples of the oligosaccharide include carbohydrates each forming two monosaccharides in the body (specifically, sucrose, maltodextrin, corn syrup and high-fructose corn syrup). Among them, important oligosaccharides are disaccharides, of which sucrose, which is known as cane sugar or beet sugar, is preferred. Examples of the complex polysaccharide include maltodextrin.
Examples of preferred carbohydrate sweeteners include a fructose-glucose mixture, which serves as an energy source and provides required calories, and carbohydrate such as sucrose, which forms glucose and fructose through hydrolysis in the gastrointestinal tract.
The carbohydrates include a natural carbohydrate present in fruit juice or in the tea extract, and an intentionally added carbohydrate. Carbohydrate derivatives, polyhydric alcohols (e.g., glycerols), and artificial sweeteners may also be employed in the aforementioned beverage, since they provides a sweeter which is readily adsorbed by the body so as to provide the whole body with energy.

Examples of the source of sodium ions (C) include a readily available sodium salt such as sodium chloride, sodium carbonate, sodium hydrogencarbonate, sodium citrate, sodium phosphate, sodium hydrogenphosphate, sodium tartrate, sodium benzoate or a mixture thereof. Sodium ions may be derived from a fruit juice or a tea extract which is added to the beverage.

Examples of the source of potassium ions (D) include potassium salts such as potassium chloride, potassium carbonate, potassium sulfate, potassium acetate, potassium hydrogencarbonate, potassium citrate, potassium phosphate, potassium hydrogenphosphate, potassium tartrate, potassium sorbate, and a mixture thereof. Potassium ions may be derived from a fruit juice or a tea extract which is added to the beverage.

In addition to sodium ions and potassium ions, the aforementioned beverage may further contain chloride ions in amount of 0.001 to 0.5 wt.%. The amounts are preferably 0.002 to 0.4 wt.%, more preferably 0.003 to 0.3 wt.%. Chloride ions may be supplied from a chloride salt such as sodium chloride or potassium chloride. The aforementioned beverage may further contain ions of trace elements such as calcium, magnesium, zinc, and iron. These ions may also be supplied in the form of salt. The ions present in the beverage include intentionally added ions and ions intrinsically present in the beverage.

The container-packed beverage preferably contains a bitterness-reducer for making the beverage to be drunk more smoothly. The bitterness-reducer is preferably cyclodextrin. Examples of the cyclodextrin employed in the beverage include α-, β-, and γ-cyclodextrin and branched α-, β- , and γ-cyclodextrin. The amount of cyclodextrin in the beverage is preferably 0.005 to 0.5 wt.%, more preferably 0.01 to 0.3 wt.%.
To the aforementioned container-packed beverages, there may be added an additional component which can be used with a tea-originating ingredient. Examples of such an additive include an anti-oxidant, a flavor, an ester, an organic acid, an organic acid salt, an inorganic acid, an inorganic acid salt, an inorganic salt, a dye, an emulsifier, a preservative, a seasoning agent, a sweetener, a sour agent, gum, an emulsifier, oil, a vitamin, an amino acid, a fruit extract, a vegetable extract, a pollen load extract, a pH-adjuster, and a quality-stabilizer. These additives may be used singly or in combination.

In order to improve the taste of the aforementioned beverage, a flavor or a fruit juice may be added to the beverage. Natural and synthetic flavors and fruit juices may be employed. These additives may be selected from among fruit juices, fruit flavors, plant flavors, and mixtures thereof. A combination of a fruit juice and a tea flavor, preferably a green tea or black tea flavor is preferred in terms of a taste. Examples of preferred fruits juice include a juice from apple, pear, lemon, lime, mandarin, grape fruit, cranberry, orange, strawberry, grape, kiwi fruit, pineapple, passion fruit, mango, guava, raspberry and cherry. Among them, citrus juice (preferably, grape fruit, orange, lemon, lime or mandarin juice), mango juice, passion fruit juice, guava juice, and a mixture thereof are preferred. Examples of preferred natural flavors include those obtained from jasmine, chamomile, rose, peppermint, hawthorn, chrysanthemum, water chestnut, sugar cane, *Mannen*-*take* (Reishi Mushroom), and bamboo shoot.
The amount of fruit juice incorporated into the aforementioned beverage is preferably 0.001 to 20 wt.%, more preferably 0.002 to 10 wt.%. A fruit flavor, a plant flavor, a tea flavor and a mixture thereof may be used. Examples of more preferred flavors employed in the beverage include citrus flavors such as orange flavor, lemon flavor, lime flavor and grape fruit flavor. Other fruit flavors such as apple flavor, grape flavor, raspberry flavor, cranberry flavor, cherry flavor and pineapple flavor may also be employed. These flavors may be made from a natural product such as fruit juice or essential oil, or a synthetic product. Examples of the flavor also include a variety of blends of flavors, for example, a mixture of lemon flavor, lime flavor, citrus flavor and selected spices (i.e., a typical flavor for cola drink). Examples of flavors in the form of oleophilic concentrate or extract which may be incorporated into the beverage include synthetic flavoring esters, alcohols, aldehydes, terpenes, and sesqui-terpenes. The beverage of the present invention preferably contains such a flavor in an amount of 0.0001 to 5 wt.%, more preferably 0.001 to 3 wt.%.

The aforementioned beverage may further contain a sour agent, which is employed so as to maintain the pH of the beverage of the present invention to 2 to 6. A dissociative acid or a sodium salt or a potassium salt of an acid may be employed. Examples of preferred acids include organic acids and inorganic acids which can be used in foods. Preferred examples include citric acid, malic acid, fumaric acid, adipic acid, gluconic acid, tartaric acid, ascorbic acid, acetic acid, phosphoric acid, and mixtures thereof. Of these, citric acid and malic acid are more preferred. These sour agents also serve as an anti-oxidant for stabilizing ingredients of the beverage. Examples of the anti-oxidant other than these sour agents include ascorbic acid and plant extracts.

The aforementioned beverage may further contain a vitamin. Examples of preferred vitamins include vitamin A, vitamin C, and vitamin E. Other than vitamins A, C, and E, other vitamins such as vitamin D and vitamin B may also be employed. The beverage of the present invention may also contain minerals. Preferred examples of preferred minerals include calcium, chromium, copper, fluorine, iodine, iron, magnesium, manganese, phosphorus, selenium, silicon, molybdenum and zinc. Of these, magnesium, phosphorus, and iron are more preferred.

### B) Tea-based container-packed beverage

A tea-based container-packed beverage containing non-polymeric catechins ((A) non-epi-catechin and (B) epicatechin), with the following composition:
(i) (A) + (B) = 50 to 2,500 mg,
(ii) (A) = 5 to 2,250 mg, and
(iii) (A)/(B) = 0.1 to 9.0, based on the container-packed beverage (500 mL).

In the above beverage, a portion (30 to 98 wt.%, preferably 40 to 90 wt.%) of the catechins is preferably selected from among epigallocatechin gallate, gallocatechin gallate, epigallocatechin and gallocatechin. In this case, the beverage is very tasty, and a long-lasting astringent taste is reduced, which is preferred. The beverage contains at least one species selected from among epigallocatechin gallate, gallocatechin gallate, epigallocatechin and gallocatechin. However, the beverage generally contains all catechin species.

In order to maintain the color tone of the aforementioned beverage for a long period of time, the beverage preferably has a non-epi-catechin/epi-catechin ratio of 0.1 to 9.0, more preferably 0.5 to 9.0, even more preferably 0.67 to 9.0, yet even more preferably 1.0 to 9.0.
The beverage has preferably has a pH (at 25°C) of 3 to 7, more preferably 4 to 7, more preferably 5 to 7, from the viewpoints of taste and chemical stability of catechins.

To the aforementioned container-packed beverages, there may be added an additional component which can be used with a tea-originating ingredient. Examples of such an additive include an anti-oxidant, a flavor, an ester, an organic acid, an organic acid salt, an inorganic acid, an inorganic acid salt, an inorganic salt, a dye, an emulsifier, a preservative, a seasoning agent, a sweetener, a sour agent, a fruit extract, a vegetable extract, a pollen load extract, a pH-adjuster and a quality-stabilizer. These additives may be used singly or in combination.
Examples of the sweetener include sucrose, glucose, fructose, high fructose corn syrup, glycyrrhizin, stevia, aspartame, fructo-oligosaccharide, galacto-oligosaccharide, and other oligosaccharides such as cyclodextrin. Examples of the cyclodextrin include α-, β-, and γ-cyclodextrin and branched α-, β-, and γ-cyclodextrin.
Examples of the sour agent include natural fruit juice, citric acid, tartaric acid, malic acid, lactic acid, fumaric acid and phosphoric acid.
Examples of the inorganic acid and inorganic acid salt include phosphoric acid, disodium phosphate, sodium metaphosphate and sodium polyphosphate. Examples of the organic acid and organic acid salt include citric acid, succinic acid, itaconic acid, malic acid and sodium citrate.

The container used for receiving the container-packed beverages may be a molded container formed mainly of polyethylene terephthalate (a so-called PET bottle), which is widely employed for general beverages. Also, generally employed containers such as a metal can, a paper composite container with metal foil or plastic film and a bottle may also be used for providing the beverages. The term "container-packed beverage(s)" employed herein refers to a beverage which can be drunk without dilution.
When the container is formed of a material (e.g., metal can) which allows heat sterilization after packing of the beverage, the beverage filled in the container is sterilized under the conditions as stipulated by the Food Sanitation Law. When a containers which cannot be subjected to retort sterilization such as a PET bottle or a paper container is employed, in one possible procedure, the beverage is sterilized at high temperature for a short time under the same sterilization conditions as employed above by means of, for example, a plate heat-exchanger, then cooled to a predetermined temperature, and filled into the container. Alternatively, to the container in which a content is placed under sterilized conditions, other ingredients may be added. Yet alternatively, the content heat-sterilized under acidic conditions may be returned to a neutral pH under sterilized conditions, or the content heat-sterilized under neutral conditions may be returned to an acidic pH under sterilized conditions.

The catechin content in the agent for improving muscle force and etc. of the present invention, which varies depending on applications, is generally 0.01 to 5 mass%, preferably 0.05 to 5 mass%, more preferably 0.1 to 1 mass%, in the case of foods, pet foods, etc. In the cases of products other than those mentioned above (e.g., oral solid products such as tablets, granules, and capsules; and oral liquid products such as oral liquid and syrup), the catechin content is generally 0.01 to 95 mass%, more preferably 5 to 95 mass%, even more preferably 10 to 95 mass%.

The daily dose (as an amount of catechin) of the agent for improving muscle force and etc. of the present invention (effective dose), which varies depending on the intensity of physical exercise, is preferably 100 to 3,000 mg/60 kg-body weight, more preferably 250 to 2,000 mg/60 kg-body weight, even more preferably 250 to 1,000 mg/60 kg-body weight.
The dosing period, which varies depending on the intensity of physical exercise, is preferably 3 to 5 days or longer, more preferably 1 to 2 weeks or longer, even more preferably 3 to 8 weeks or longer.

### Examples

The present invention will be described hereinbelow in detail by way of Test Examples employing agents falling within the scope of the present invention, followed by preparation examples of the agent for improving muscle force and etc. of the invention described as Examples.

### Test Example 1: An agent for improving muscle force (Effect of catechins on improving muscle force in mice)

In Test Example 1, a green tea extract having a total catechin content of 81% was employed. The compositions of catechin species are shown in Table 1.

### Analysis of catechins

Each catechin agent was dissolved in water, and the solution was filtered through a filter (0.8 µm). The thus-obtained sample was subjected to liquid chromatography employing an octadecyl group-introduced packed column for liquid chromatography, L-Column TM ODS (φ: 4.6 mm × 250 mm, product of Chemicals Evaluation and Research Institute, Japan). Compounds contained in the sample were detected at a column temperature of 35°C through the gradient method at a wavelength of 280 nm. The gradient mobile phase employed in liquid chromatography included liquid A (0.1 mol/L aqueous acetic acid) and liquid B (0.1 mol/L acetic acid solution in acetonitrile).

**[Table 1]**

| | | Proportions (%) |
|---|---|---|
| Non-epi-catechins | catechin + catechin gallate | 4 |
| | gallocatechin | 7 |
| | gallocatechin gallate | 4 |
| | (subtotal) | 15 |
| Epi-catechins | epicatechin | 9 |
| | epicatechin gallate | 12 |
| | epigallocatechin | 23 |
| | epigallocatechin gallate | 41 |
| | (subtotal) | 85 |
| Epi-catechins/ all catechins (%) | | 15 |
| Non-epi/epi | | 0.18 |

Male Balb/c mice (6 weeks old) were bred for one week for adaptation and divided into four groups on the basis of the body weight (control group, catechin group, exercise group, and exercise plus catechin group) (each group: n=5). Thereafter, mice of these groups were given diets having compositions shown in Table 2 for eight weeks.

**[Table 2]**

| Diet composition (wt.%) | | | | |
|---|---|---|---|---|
| | Control group | Catechin group | Exercise group | Exercise +catechin group |
| Casein | 20 | 20 | 20 | 20 |
| DL-methionine | 0.2 | 0.2 | 0.2 | 0.2 |
| Fats/oils | 10 | 10 | 10 | 10 |
| α-Potato starch | 55.5 | 55 | 55.5 | 55 |
| Cellulose | 8.1 | 8.1 | 8.1 | 8.1 |
| Minerals | 4 | 4 | 4 | 4 |
| Vitamins | 2.2 | 2.2 | 2.2 | 2.2 |
| Catechin formulation | | 0.5 | | 0.5 |
| Total | 100 | 100 | 100 | 100 |

During breeding for eight weeks, the mice of the exercise group were subjected to a treadmill running exercise at 20 m/min for 30 minutes three times per week. The mice of the exercise plus catechin group were given a catechin-containing diet and subjected to the treadmill running exercise three times per week. The mice of the control group and the catechin group were not subjected to a treadmill running exercise.

### Measurement of muscle force of removed muscle

After breeding for eight weeks, soleus muscle was removed from a mouse of each group and fixed to a transducer (FORT100, product of WPI) in 37°C Krebs solution (aeration: 95%-oxygen, 5%-carbon dioxide)). Subsequently, under electrical stimulation (0.2 msec, 40 Hz), the maximum muscle force was determined.

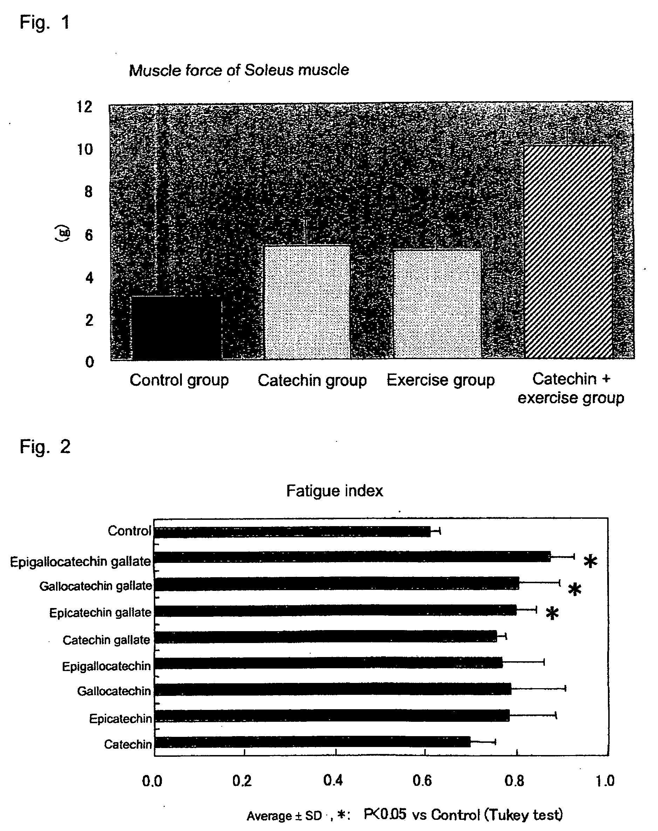

Figure 1 shows the results of soleus muscle force measurement.

As is clear from Figure 1, intake of catechin enhanced muscle force of mouse under non-exercise breeding, indicating that catechin is effective for improving muscle force. Physical exercise without intake of catechin also enhanced muscle force. However, a combination of physical exercise and intake of catechin more effectively enhanced muscle force.
Thus, catechin has an effect of enhancing the muscle force-improving effect of exercise, and is useful as an agent for improving exercise effect which can make the effect of physical exercise more effective. When intake of catechin and physical exercise are employed in combination, muscle force and exercise effect can be more improved.

### Test Example 2: Electrical stimulation of isolated muscle

Mice were bred in a similar manner to that for the control group of Test Example 1. Thereafter, soleus muscle was removed from each mouse.
The isolated soleus muscle was fixed to a transducer (FORT100, product of WPI) in 37°C Krebs solution (aeration: 95%-oxygen, 5%-carbon dioxide). The effect of catechins on the isolated muscle was analyzed. Specifically, each of eight catechin species (catechin, epicatechin, gallocatechin, epigallocatechin, catechin gallate, epicatechin gallate, gallocatechin gallate, and epigallocatechin gallate) was dissolved in a Krebs solution to a concentration of 50 µM. Each muscle piece was equilibrated in each solution for three minutes, and electrically stimulated (330 ms/2-sec). The frequency of stimulation was 40 Hz. Figure 2 shows muscle forces after 1 minute stimulation as fatigue indices, each of which is represented as the ratio to muscle forces at the start of electrical stimulation defined as 1. A Krebs solution to which no catechin species had been added was employed as a control solution.

As shown in Figure 2, eight catechin species more effectively inhibited decrease in muscle force caused by electrical stimulation compared with the control solution. Among catechin species, catechin provided the lowest muscle fatigue resistance, while, in particular, epicatechin gallate, gallocatechin gallate, and epigallocatechin gallate improved muscle fatigue resistance.

### Production Examples 1 to 6 and Comparative Examples 1 to 6

In each Example, ingredients shown in Table 3 or 4 were mixed, and ion-exchange water was added to the mixture so as to adjust the total volume of the resultant mixture, to thereby prepare a liquid mixture. The liquid mixture was subjected to sterilization and hot-pack filling in accordance with the Food Sanitation Law, whereby a container-packed beverage was produced. Thus, a container-packed non-tea-based beverage for improving muscle force or exercise effect was produced.

**[Table 3]**

| (g) | | | | | | |
|---|---|---|---|---|---|---|
| Formulation | Prodn. Ex. 1 | Prodn. Ex. 2 | Prodn. Ex. 3 | Prodn. Ex. 4 | Prodn. Ex. 5 | Prodn. Ex. 6 |
| Green tea extract A | 1 | 0.3 | 1 | 1 | | 4 |
| Green tea extract B | | | | | 0.08 | |
| Green tea extract C | | | | | 0.2 | |
| Ascorbic acid | 0.03 | 0.03 | 0.03 | 0.03 | 0.03 | 0.03 |
| Citric acid | 0.2 | 0.2 | 0.2 | 0.2 | 0.2 | 0.2 |
| 3Na citrate | 0.1 | 0.1 | 0.1 | 0.1 | 0.1 | 0.1 |
| Fructose | | | 2 | | | |
| Glucose | | | | 2 | 2 | 7 |
| Artificial sweetener | 5 | 5 | 3 | 3 | 3 | 5 |
| Na chloride | 0.05 | 0.05 | 0.05 | 0.05 | 0.05 | 0.05 |
| K chloride | 0.02 | 0.02 | 0.02 | 0.02 | 0.02 | 0.02 |
| Flavor | 0.1 | 0.1 | 0.1 | 0.1 | 0.1 | 0.1 |
| Ion-exchange water | bal. | bal. | bal. | bal. | bal. | bal. |
| Total amount | 100 | 100 | 100 | 100 | 100 | 100 |
| pH of beveraqe | 3.5 | 3.5 | 3.5 | 3.5 | 3.4 | 3.5 |
| Long-term taste | A | A | A | A | A | B |
| Stability of bitterness and astringency | A | A | A | A | A | A |
| Smoothness in drinking | A | A | A | A | A | A |
| Stability of color tone | A | A | A | A | A | B |

**[Table 4]**

| (g) | | | | | | |
|---|---|---|---|---|---|---|
| Formulation | Comp. Ex. 1 | Comp. Ex. 2 | Comp. Ex. 3 | Comp. Ex. 4 | Comp. Ex. 5 | Comp. Ex.6 |
| Green tea extract A | 1 | | 1 | 1 | 1 | |
| Green tea extract B | | | | | | 0.35 |
| Green tea extract C | | 0.23 | | | | |
| Ascorbic acid | | 0.03 | 0.03 | 0.03 | 0.03 | 0.03 |
| Citric acid | | 0.2 | 0.2 | 0.2 | 0.2 | 0.2 |
| 3Na citrate | 0.33 | 0.1 | 0.1 | 0.1 | 0.1 | 0.1 |
| Fructose | | | | | | |
| Glucose | | | | | 22 | |
| Artificial sweetener | 5 | 5 | 5 | 5 | 3 | 5 |
| Na chloride | 0.05 | 0.05 | 2.5 | 0.05 | 0.05 | 0.05 |
| K chloride | 0.02 | 0.02 | 0.02 | 0.9 | 0.02 | 0.02 |
| Flavor | 0.1 | 0.1 | 0.1 | 0.1 | 0.1 | 0.1 |
| Ion-exchanqe water | bal. | bal. | bal. | bal. | bal. | bal. |
| Total amount | 100 | 100 | 100 | 100 | 100 | 100 |
| pH of beverage | 6.5 | 3.5 | 3.5 | 3.5 | 3.5 | 3.5 |
| Long-term taste | B | C | D | D | C | C |
| Stability of bitterness and astringency | D | C | C | C | C | D |
| Smoothness in drinking | D | B | D | D | D | D |
| Stability of color tone | D | B | C | C | D | D |

### Green tea extract A

POLYPHENON HG (green tea extract concentrate, product of Tokyo Food Techno Co., Ltd.) (100 g) was suspended in 95% aqueous ethanol solution (490.9 g) at ambient temperature under stirring at 250 rpm. Subsequently, Kuraray Coal GLC (activated carbon, product of Kuraray Chemical Co., Ltd.) (20 g) and Mizuka Ace #600 (acid clay, product of Mizusawa Industrial Chemicals Ltd.) (35 g) were added to the suspension, and the mixture was continuously stirred for about 10 minutes. Another 40% aqueous ethanol solution (409.1 g) was added dropwise to the above mixture over 10 minutes, and the product was stirred at room temperature for about 30 minutes. Thereafter, the resultant mixture was filtered through a filter paper (No. 2), to thereby remove activated carbon and precipitates, and the filtrate was further filtered through a 0.2-µm membrane filter. Finally, ion-exchange water (200 g) was added to the re-filtered liquid, and ethanol contained in the liquid was removed at 40°C and 0.0272 kg/cm², whereby green tea extract A was produced.
The product was found to have a non-polymeric catechin content of 22 mass%.

### Green tea extract B

A green tea extract concentrate, having a non-polymeric catechin content of 33.70 mass% and a gallate form content of 50.7 mass%.

### Green tea extract C

A green tea extract concentrate, having a non-polymeric catechin content of 81.40 mass% and a gallate form content of 60.5 mass%.

### Production Examples 7 to 10

Components shown in Table 5 were mixed, and the required treatments were performed, to thereby produce tea-based container-packed beverages for improving muscle force or exercise effect.

**[Table 5]**

| (g) | | | | | | |
|---|---|---|---|---|---|---|
| | | | Prodn. Ex. 7 | Prodn. Ex. 8 | Prodn. Ex. 9 | Prodn. Ex. 10 |
| Tea extract | Oolong tea extract^{*1} | | - | - | - | 947 |
| | Tea extract concentrate^{*2} A | | 4.4 | - | - | - |
| | Tea extract concentrate^{*2} B | | - | - | 4.8 | - |
| | Tea extract concentrate^{*2} C | | - | - | - | 17.6 |
| | Tea extract concentrate^{*2} D | | - | 4.2 | - | - |
| Na ascorbate | | | 0.3 | 0.3 | 0.39 | 2.2 |
| Distilled water | | | bal. | bal. | bal. | bal. |
| Total mass | | | 1,000 | 1,000 | 1,300 | 4,400 |
| pH^{*3} | | | 6.0 | 6.0 | 3.8 | 6.5 |
| Post-treatment | | temp. (°C) | 121 | - | 65 | 139 |
| (heating) | | time (min) | 5 | - | 10 | *4 |

| | | | | | | |
|---|---|---|---|---|---|---|
| *1) Produced by adding oolong tea leaves (33 g) to ion-exchange water (1 kg) maintained at 85°C, performing extraction for 8 minutes, and filtering the extract through a flannel filter with cooling by means of a heat exchanger. *2) Tea extract concentrates A) Catechin content 33%, non-epi-catechin content 4% (product of Mitsui Norin Co., Ltd.) B) Catechin content 33%, non-epi-catechin content 14% C) Catechin content 30%, non-epi-catechin content 3% (product of Mitsui Norin Co., Ltd.) D) Catechin content 30%, non-epi-catechin content 14% *3) Adjusted with citric acid/disodium phosphate (Production Examples 7 and 8), with citric acid (Production Example 9), and with sodium hydrogencarbonate (Production Example 10) *4) 10 seconds (degassed before sterilization) | | | | | | |

The beverages produced in Production Examples 7 to 10 favorably exhibited no transparency change over time and had a stable color tone.

### Production Examples 11 to 13

In Production Examples 11 to 13, tea leaves (100 g) as shown in Table 6 were extracted with distilled water (1,000 g) at 80°C for 10 minutes in a clean bench. The extract was filtered, to thereby produce a tea extract. Subsequently, each of the below-described compositions were mixed, degassed and heated at 139°C for 10 seconds. The product was packed in a 500-mL PET bottle, whereby a tea-based beverage for improving muscle force or exercise effect was produced.

**[Table 6]**

| (g) | | | | |
|---|---|---|---|---|
| Composition | | Prodn. Ex. 11 | Prodn. Ex. 12 | Prodn. Ex. 13 |
| Tea extract (liquid) | | Green tea 950 | Black tea 935 | Oolong tea 950 |
| Tea extract^{*1} | Tea extract concentrate C | 9.0 | 23.3 | - |
| | Tea extract concentrate D | - | - | 9.0 |
| Na ascorbate | | 1.8 | 2.5 | 1.8 |
| ion-exchange water | | bal. | bal. | bal. |
| Total mass | | 4,500 | 5,000 | 4,500 |
| Non-epi catechins (mg/500 mL) | | 354 | 298 | 408 |
| Catechins (mg/500 mL) | | 835 | 861 | 816 |
| pH^{*2} | | 6 | 6 | 6 |

| | | | | |
|---|---|---|---|---|
| *1) Tea extract concentrates C) Catechin content 30%, non-epi-catechin content 3% (product of Mitsui Norin Co., Ltd.) D) Catechin content 30%, non-epi-catechin content 14% *2) pH adjusted with sodium hydrogencarbonate | | | | |

The container-packed beverages produced in Production Examples 11 to 13 exhibited virtually no changes in color tone stability or transparency during storage. The beverages were easy to drink and very tasty.

## Claims

1. Non-medical use of catechins as effective ingredient in combination with physical exercise for improving muscle force, wherein the use of a dietary supplement comprising:
- a source of at least one of epigallocatechin gallate (EGCG), epicatechin gallate (ECG), epicatechin (EC), tannic acid or related catechins; and
- a source of Gypenosides
is excluded.

2. Non-medical use of catechins as effective ingredient for increasing the effect of physical exercise on the enhancement of muscle force, wherein the use of a dietary supplement comprising:
- a source of at least one of epigallocatechin gallate (EGCG), epicatechin gallate (ECG), epicatechin (EC), tannic acid or related catechins; and
- a source of Gypenosides
is excluded.

3. Non-medical use according to claim 1 or 2, wherein the catechins are selected from the group consisting of epicatechin gallate, gallocatechin gallate and epigallocatechin.

## Patentansprüche

1. Nicht-medizinische Verwendung von Catechinen als Wirkstoff in Kombination mit körperlicher Übung zum Verbessern der Muskelkraft, worin die Verwendung eines Nahrungsergänzungsmittels, umfassend:
- eine Quelle von mindestens einem aus Epigallocatechingallat (EGCG), Epicatechingallat (ECG), Epicatechin (EC), Taninsäure oder verwandten Catechinen; und
- eine Quelle von Gypenosiden
ausgenommen ist.

2. Nicht-medizinische Verwendung von Catechinen als Wirkstoff als Erhöhen der Wirkung von körperlicher Übung auf die Verbesserung von Muskelkraft, worin die Verwendung eines Nahrungsergänzungsmittels, umfassend:
- eine Quelle von mindestens einem aus Epigallocatechingallat (EGCG), Epicatechingallat (ECG), Epicatechin (EC), Taninsäure oder verwandten Catechinen; und
- eine Quelle von Gypenosiden
ausgenommen ist.

3. Nicht-medizinische Verwendung gemäß Anspruch 1 oder 2, worin die Catechine aus der Gruppe bestehend aus Epicatechingallat, Gallocatechingallat und Epigallocatechin ausgewählt sind.

## Revendications

1. Utilisation non-médicale de catéchines comme ingrédient efficace en combinaison avec de l'exercice physique pour améliorer la force musculaire, où l'utilisation d'un complément alimentaire comprenant :
une source d'au moins l'un du gallate d'épigallocatéchine (EGCG), du gallate d'épicatéchine (ECG), de l'épicatéchine (EC), de l'acide tannique ou de catéchines connexes ; et
une source de Gypénosides
est exclue.

2. Utilisation non-médicale de catéchines comme ingrédient efficace pour augmenter l'effet de l'exercice physique sur l'amélioration de la force musculaire, où l'utilisation d'un complément alimentaire comprenant :
une source d'au moins l'un du gallate d'épigallocatéchine (EGCG), du gallate d'épicatéchine (ECG), de l'épicatéchine (EC), de d'acide tannique ou de catéchines connexes ; et
une source de Gypénosides
est exclue.

3. Utilisation non-médicale selon la revendication 1 ou 2, dans laquelle les catéchines sont choisies dans le groupe constitué de gallate d'épicatéchine, de gallate de gallocatéchine et d'épigallocatéchine.
